Europäisches Patentamt

European Patent Office

Office européen des brevets

(11) Veröffentlichungsnummer: **0 214 933**
A2

(12) # EUROPÄISCHE PATENTANMELDUNG

(21) Anmeldenummer: 86810387.0

(22) Anmeldetag: 28.08.86

(51) Int. Cl.⁴: **A 61 K 45/06**
A 61 K 31/47, A 61 K 31/49
A 61 K 31/16
//(A61K31/49, 31:47, 31:16)

(30) Priorität: 03.09.85 CH 3784/85

(43) Veröffentlichungstag der Anmeldung:
18.03.87 Patentblatt 87/12

(84) Benannte Vertragsstaaten:
BE CH DE FR GB IT LI LU NL SE

(71) Anmelder: CIBA-GEIGY AG
Klybeckstrasse 141
CH-4002 Basel(CH)

(72) Erfinder: Jung, Albrecht, Dr.
Hausserstrasse 142
D-7400 Tübingen(DE)

(72) Erfinder: Stahel, Erich, Dr.
7, rue du Banquier
F-75013 Paris(FR)

(72) Erfinder: Vosbeck, Klaus Dieter, Dr.
13049 Caminito Mar Villa
Del Mar California 92014(US)

(54) Kombinationspräparate zur Behandlung von Malaria.

(57) Humane Malaria, insbesondere Malaria tropica, kann erfolgreich durch kombinierte Verabreichung von einem Eisen(III)-Chelator der Klasse umfassend Desferrioxamin B und Desferriferrithiocin, sowie ihre pharmakologisch wirksamen Derivate, als Komponente A einerseits und einem konventionellen Antimalarikum der Kategorie von Schizontoziden als Komponente B andererseits in synergistischem Verhältnis behandelt werden. Die Therapie ist besonders vorteilhaft zur Bekämpfung von Chloroquin-resistenten Stämmen von Plasmodium falciparum.

EP 0 214 933 A2

0214933

CIBA-GEIGY AG                           4-15488/=

Basel (Schweiz)


Kombinationspräparate zur Behandlung von Malaria
_____


Malaria ist allgemein bekannt als eine gefährliche Infektionskrankheit, welche durch gewisse Stechmückenarten (Moskitos) der
Gattung Anopheles übertragen wird und sich durch periodisch wiederholte, mit Schüttelfrost verbundene Hochfieberanfälle von 40-41°C
manifestiert. Malaria ist in verschiedenen Formen weltweit verbreitet in feuchtwarmen Gebieten, insbesondere jenen der subtropischen und tropischen Zonen, und ihre derzeitige Ausbreitung
wird auf etwa 200-400 Millionen Fälle geschätzt. Allein in Afrika
rechnet man bei Malaria-Erkrankungen in etwa 10 % der Fälle mit
direktem tödlichem Ausgang; in den übrigen Fällen hat sie eine
beträchtliche langdauernde Schwächung zur Folge. Bei Kleinkindern
wird sie als Ursache in etwa 50 % aller Todesfälle verantwortlich
gehalten. Jahrzehnte, sogar Jahrhunderte trotzt die Seuche allen
Versuchen um ihre Bannung und stellt nach wie vor eines der grossen
Probleme des Weltgesundheitswesens dar. Abgesehen von grossangelegten umfassenden Bemühungen um gesamthafte oekologische Lösung
(wie Bekämpfen der Stechmücken durch Insekticide oder, noch tiefgreifenden, durch Entwässern und Austrocknen der Sümpfe), stehen im
Vordergrund des Interesses die Möglichkeiten der medizinischen
Behandlung des Menschen als Träger, darunter auch insbesondere die
Suche nach therapeutischen Mitteln zur unmittelbaren Bekämpfung der
Krankheitserreger.


In der Tat ist Malaria eine Gesamtbezeichnung für mehrere Krankheiten, die mit ähnlichem Krankheitsbild verlaufen, aber deren
Erreger - parasitäre Protozoen aus der Klasse Sporentierchen - verschiedene Arten von Plasmodium sind. Diese Erkrankungen treten nicht nur beim Menschen, sondern auch bei Säugetieren auf, wobei sie Art-spezifisch sind, d.h. von einer Tierart auf eine andere oder auf Menschen nicht übertragen werden können. Beim Menschen unterscheidet man dem Krankheitsbild nach 3 Formen: Malaria tertiana (Erreger Plamodium vivax und P. ovale), M. quartana (P. malariae) und die besonders schwere und bösartige M. tropica (P. immdaculatum oder falciparum). Die medizinische Behandlung und Medikamente sind allerdings in allen 3 Fällen dieselben.

Charakteristisch für alle Plasmodien-Arten ist ihr komplizierter Entwicklungszyklus, dessen geschlechtliche Vermehrungsstadien im Darmtrakt weiblicher Stechmücken (als Ueberträger) sich abspielen und in ihrer Speicheldrüse zur Uebertragung bereitgehalten werden, wogegen die enorme ungeschlechtliche Vermehrung in der Leber und den roten Blutkörperchen des Menschen als Träger stattfindet. Auf den Menschen wird die Infektion beim Mückenstich übertragen, wobei die winzigen einkernigen Sichelkeime (Sporozoiten) des Parasiten mit dem Speichel des Ueberträgers eingeimpft werden und auf dem Blutwege in die Leber des Trägermenschen gelangen. Nach Eindringen in die Leberzelle wächst ungeschlechtlich der Sporozoit zu einem vielkernigen Gebilde (Schizonten) heran, durch dessen Zerfall etwa 5000-10 000 einkernige Sprosszellen (Merozoiten) freigesetzt werden. Diese können zur weiteren Fortpflanzung wiederum die Leberzellen befallen, oder die Erythrozyten (rote Blutkörperchen) angreifen. Jeder Merozoit dringt in ein Blutkörperchen ein, baut dessen Zellmasse weitgehend ab und verwendet die gewonnenen Bausteine - vorwiegend Aminosäuren - für eigene Stoffwechselprozesse und Energiegewinnung. Dadurch bildet er in einem extram raschen ungeschlechtlichen Vermehrungsvorgang (Schizogonie) innerhalb von 48 (bzw. 72) Stunden 10-20 neue Merozoiten und zerstört dabei völlig die Wirtszelle. (Mit derartigen Wachstumsraten übertreffen Plasmodien selbst schnellwachsende Tumoren.) Die entstandenen Merozoiten befallen weitere Erythrozyten zu einem neuen Vermehrungsstadium. Dieser zyklische Verlauf ist für die wohlbekannten Malaria-Symptome verantwortlich, da insbesondere die Blutzerfallsprodukte den hohen Fieber und Schüttelfrost verursachen und die typischen
(fast) fieberfreien Perioden von 48 (bei M. tertiana und M. tropica)
bzw. 72 (bei M. quartana) dem Vermehrungsvorgang des Parasiten im
Blut zeitlich entsprechen. Nach mehrmaliger Wiederholung lässt die
Intensität (nicht aber der Zeitabstand!) der Anfälle allmählich bis
zum völligen Abklingen nach. Wahrscheinlich spielt dabei einerseits
die natürliche Erschöpfung und Abnahme der Vitalität der Erregerzellen, andererseits die Entwicklung einer Art Immunität des Trägers
eine Rolle.

Wie andere intrazelluläre Parasiten, stellt auch Plasmodium für die
Therapie ein besonderes Problem dar: abgesehen von den verschiedenen
Entwicklungsformen ist der Mikroorganismus fast nie einem direktem
Angriff eines exogenen oder endogenen (d.h. durch den Träger
produzierten) Inhibitors zugänglich, da er meistenzeits in Wirtszellen Unterschlupf findet und somit durch 2 funktionell sehr
verschiedene Zellmembranen zweier physiologisch weit auseinanderliegenden Zellarten (der eigenen und derjenigen der Wirtszelle)
geschützt wird. Die funktionellen Eigenschaften der Erythrozytenmembran sperren den Zugang zum Parasiten für eine Reihe von
Inhibitoren (wie körpereigenen Immunfaktoren), welche, wenn er
extrazellulär leben würde, sicherlich gut wirksam wären. Wahrscheinlich aus diesem Grunde haben bisher auch alle Versuche gescheitert,
die Krankheit durch immunologische Behandlungsmethoden einschliesslich Schutzimpfung unter Kontrolle zu bringen. Somit bleiben
Chemotherapeutika unter den Antimalariamitteln immer noch am
erfolgreichsten, wie z.B. das klassische Chinin, welches für
Jahrhunderte konkurrenzlos das einzige wirksame Heilmittel war und
trotz gewisser Nachteile weiterhin eine bedeutende Stelle in der
Malaria-Therapie einnimmt. Im Laufe der letzten 50 Jahre wurden
zahlreiche Chemotherapeutika verschiedenster Strukturklassen als
Schizontozide entwickelt, d.h. Wirkstoffe, die in den Entwicklungszyklus der Plasmodien in einem ihrer ungeschlechtlichen Vermehrungsstadien eingreifen und die Teilung entweder im Lebergewebe oder in
Erythrocyten unterbinden, oder sogar die Merozoiten töten. Präparate
der ersten Gruppe (Gewebe-Schizontozide) sind beispielsweise durch Primaquin [6-Methoxy-8-(4-amino-1-methylbutylamino)chinolin]
repräsentiert; stellvertretend für die Gruppe der Blut-Schizontozide
ist neben Chinin auch das bisher erfolgreichste Antimalarikum
Chloroquin [7-Chlor-4-(4-diethylamino-1-methylbutylamino)chinolin]
hervorzuheben. (Eine Zusammenstellung wichtigerer bekannter Chemotherapeutika ist in der Fachliteratur zu finden, vgl. Advances in
Malaria Chemotherapy, WHO Technical Report Series 711; World Health
Organisation, Geneva, 1984; Seiten 12, 13 und 163-175.

Die allgemeine Anwendbarkeit dieser Heilmittel vermindert sich
jedoch im Laufe der Zeit infolge der ausgeprägten Fähigkeit von
Plasmodien, Resistenz gegen Chemotherapeutika zu entwickeln. Solche
Resistenz wurde bereits Anfangs des 20. Jahrhunderts beim Chinin
beobachtet. Zurzeit betrifft sie in immer steigendem Masse
Chloroquin und intensiviert somit die Suche nach neuen Lösungen.

Die heutige tiefere Einsicht in die metabolischen Vorgänge im
Entwicklungszyklus von Plasmodien lenkte Aufmerksamkeit darauf, dass
die enorm schnelle Vermehrung der Zellpopulation, sowie deren
Ueberleben, unmittelbar von einer ausreichenden Versorgung mit Eisen
abhängig sein muss. Dies gilt insbesondere für die entsprechend
hohe DNA-Synthese, in welcher das eisenhaltige Enzym Ribonucleotidreductase eine Schlüsselrolle spielt. Zur Sicherung der Vermehrung
von Plasmodien muss Eisen in die Parasitenzelle über ein geeignetes Transportsystem gelangen. Dazu muss es unter Umständen
nicht nur durch die Zellmembran des Parasiten, sondern auch, falls
dieses es dem Erythrozyten als Wirtszelle nicht entnehmen kann,
durch die phylogenetisch verschiedene Zellmembran des Wirtes. Wenn
es nun gelingt, die Verfügbarkeit des Eisens für das Enzym zu
unterbinden, z.B. durch eine feste Bindung an einen Komplexbildner,
so könnte man den Vermehrungsmechanismus zum Stillstand bringen oder
sogar die Parasitenzelle töten.

Einem derartigen Mechanismus ist offensichtlich die antibakterielle
Wirkung von gewissen aus natürlichen Quellen, insbesondere aus
Mikroorganismen, gewonnenen Eisen(III)-Chelatoren zuzuschreiben.

Eine gewisse allgemeine antibakterielle Wirksamkeit in vitro wurde z.B. beim Desferri-ferrithiocin [2-(3-Hydroxy-2-pyridyl)-4-methyl-2-thiazolin-4-carbonsäure] und seinen halbsynthetischen Derivaten der Europäischen Patentschrift 0045281, sowie beim Desferrioxamin B [6,17,28-Trihydroxy-7,10,18,21,29-pentaoxo-6,11,17,22,28-pentaaza-triacontanylamin), vgl. H. Bickel, H. Keberle und E. Vischer: Helv. Chim. Acta 46, 1385-9 [1963]. Bei Bakterien allerdings handelte es sich (abgesehen davon, dass lediglich die in vitro--Wirksamkeit nachgewiesen wurde) um den einfachen Fall, in welchem das Eisen(III)-komplexierende Mittel lediglich eine einzige, nämlich die bakterielle Membran durchdringen musste.

Im vorliegenden Fall der Parasitenzellen eines Plasmodiums hängt aber der Erfolg davon ab, ob der Komplexbildner nicht nur durch die Zellmembran des Parasiten, sondern auch durch die phylogenetisch ganz verschiedene Zellmembran des Erythrozyten als Wirtszelle transportierbar ist. Glücklicherweise ist diese Bedingung bei den oben erwähnten Eisen(III)-Chelatoren natürlichen Ursprungs und ihren halbsynthetischen Analogen erfüllt, und es konnte experimentell nachgewiesen werden, dass verschiedene Plasmodium-Arten in vitro, darunger auch Plasmodium falciparum in Kultur an menschlichen Erythrozyten, durch Desferrioxamin B nicht nur in ihrem Wachstum gehemmt, sondern auch getötet werden. Auch in Versuchen in vivo mit einer für Nagetiere spezifischen Plasmodium-Art (P. vinckei) wurde nachgewiesen, dass Desferrioxamin B bei 0,2 g/kg das Wachstum des Parasiten deutlich hemmt, und bei 1,0 g/kg ihn vollständig tötet, vgl. A. Jung et al.; Abstracts of Iron Club Meeting, Rennes (France), July 10-13, 1984. Da die Wirkung von Desferrioxamin B im Tierversuch und in der humanen Applikation bereits in einem anderen Zusammenhang korreliert wurden, und da auch ähnliche Korrelation zwischen Plasmodium vinckei in der Maus und P. falciparum im Mensch bei klassischen Antimalarika bekannt sind, sind somit auch theoretische Grundlagen gegeben, die Möglichkeit und Bedingungen für eine therapeutische Anwendung am Menschen auszuwerten. Wahrscheinlich ist man durch eine solche Analyse zum Schluss gekommen, dass die notwendige Dosierung des Heilmittels (obwohl es auch in sehr hohen Dosen toxikologisch einwandfrei ist) unpraktisch und für breitere Anwendung uninteressant wäre - jedenfalls steht fest, dass gegen therapeutische Anwendung von Desferrioxamin B oder analogen Eisenchelatoren bei humaner Malaria offensichtlich ein Vorurteil herrschte und diese nie ausprobiert worden ist.

Die ungünstige Situation hat sich durch die vorliegende Erfindung schlagartig geändert, indem sich gezeigt hat, dass die oben erwähnten Eisenchelatoren überraschenderweise gegen Chloroquinresistente Stämme von Plasmodium falciparum bei wesentlich niedrigerer Dosierung als bei normalen Stämmen wirksam sind, also gerade gegen die Parasiten, welche durch ihre Resistenz gegenüber konventionellen Chemotherapeutika als eines der schwierigsten Probleme im Kampfe gegen Malaria angesehen werden. Dabei wurde auch noch eine weitere überraschende Tatsache entdeckt, dass nämlich Eisen(III)-Chelatoren vom Typ Desferrioxamin B die Wirksamkeit der oben erwähnten Schizontozide (z.B. Chloroquin) wesentlich erhöhen, und zwar nicht nur bei Chloroquin-resistenten Plasmodium-Stämmen, sondern auch bei denjenigen mit einer normalen Empfindlichkeit gegenüber Chloroquin.

Durch einen experimentellen Vergleich der Wirksamkeit von Desferrioxamin B gegen P. falciparum in Kultur an menschlichen Erythrozyten wurde gezeigt, dass dieselbe Wachstumshemmung eines Chloroquin-resistenten Stammes im Vergleich mit einem unter gleichen Bedingungen gezüchteten, gegen Chloroquin normal empfindlichen Stamm in Abwesenheit von Chloroquin bei einer etwa dreimal niedrigeren Konzentration von Desferrioxamin B erreicht wird; die letzte halbiert sich noch weiter in Anwesenheit einer an sich unwirksamen Konzentration von Chloroquin. Ein ähnlich überraschendes Resultat wird erhalten, wenn man einen gegen Chloroquin normal empfindlichen Stamm von P. falciparum mit einer Kombination von Chloroquin und Desferrioxamin B behandelt; beide Wirkstoffe werden in Konzentrationen eingesetzt, welche, jede für sich, eine 20%ige Wachstumshemmung hervorbringen würden. Bei kombinierter Einwirkung wird eine Wachstumshemmung von 70-100 % erzielt, womit der überraschende synergistische Effekt beider Komponenten klar bewiesen wird.

Die Erfindung betrifft ein Kombinationspräparat bestehend aus 2 Komponenten in einem synergistisch wirkenden Verhältnis, und zwar aus einem Eisen(III)-Chelator der Klasse umfassend Desferrioxamin B und Desferriferrithiocin, sowie ihre pharmakologisch wirksamen Derivate als Komponente A einerseits, und einem konventionellen Antimalarikum der Kategorie von Schizontoziden als Komponente B andererseits, welche jede in einer separaten, oder beide zusammen in einer gemeinsamen pharmazeutischen Zusammensetzung, gegebenenfalls unter Beimischung von mindestens einem pharmazeutischen Trägermaterial vorliegen. Bevorzugt ist dabei eine pharmazeutische Zusammensetzung, die die beiden oben definierten Komponenten nebeneinander und gegebenenfalls auch zusammen mit konventionellen pharmazeutischen Trägermaterialien enthält. Die Erfindung betrifft auch das Verfahren zur Herstellung eines solchen oben definierten Kombinationspräparats bzw. pharmazeutischen Zusammensetzung aus ihren Komponenten durch konventionelle pharmazeutische Verfahren, sowie Verwendung dieser Kombinationspräparate bzw. pharmazeutischen Zusammensetzungen zur Behandlung der humanen Malaria. Weiter betrifft die Erfindung auch die entsprechende therapeutische Methode zur Behandlung der humanen Malaria durch gleichzeitige oder alternierende Verabreichung, von einem Eisen(III)-Chelator der Klasse umfassend Desferrioxamin B und Desferriferrithiocin, sowie ihre pharmakologisch wirksamen Derivate einerseits und einem konventionellen Antimalarikum der Kategorie von Schizontoziden andererseits, in einem synergistisch wirkenden Verhältnis und in Mengen, die zusammen gegen Malaria-Erreger wirksam sind, wobei die Verabreichung jeder Komponente in einer individuellen Darreichungsform oder beider Komponenten als Kombinationspräparat oder zusammen in Form einer gemeinsamen pharmazeutischen Zusammensetzung, insbesondere der oben definierten, erfolgt.

Unter den zu behandelnden Malaria-Erkrankungen sind Infektionen durch Plasmodien-Arten zu verstehen, z.B. durch Stämme von P. vivax oder P. ovale (M. tertiana), P. malariae (M. quartana) und insbesondere P. falciparum oder P. immaculatum (M. tropica), sowie gemischte Infektionen mit 2 oder mehreren Stämmen derselben oder einer anderen Plasmodium-Art oder auch Reinfektionen mit demselben Stamm, jedoch in einer zeitlich verschobenen Phase. Insbesondere betreffen alle oben angegebenen Aspekte der Erfindung die Behandlung von Malaria tropica mit Plasmodium falciparum als Erreger. Die oben angegebenen Aspekte betreffen auch insbesondere die Behandlung von Malaria-Erkrankungen durch Plasmodium-Stämme, welche gegen die konventionellen Chemotherapeutika, wie insbesondere Chinin und vor allem Chloroquin, sowie andere wirkungsanaloge Heilmittel, resistent sind, und darunter ganz besonders die Behandlung der Krankheit in ihrer akuten Phase, nämlich während der Vermehrung von Plasmodien in Erythrozyten des Trägers.

Die erfindungsgemäss anzuwendenden Antimalarika der Kategorie von Schizontoziden sind allgemein bekannt als konventionelle Chemotherapeutika, welche zur Wachstumshemmung bzw. Tötung der Merozoiten (der ungeschlechtlichen Wachstumsform von Plasmodien) insbesondere in humanen Erythrozyten verwendet werden. Zu dieser Kategorie gehört in erster Linie Chloroquin [7-Chlor-4-(4-diethylamino-1-methylbutylamino)-chinolin] in Form von freier Base oder einem pharmazeutisch brauchbaren Säureadditionssalz, wie insbesondere Diphosphat und Sulfat, und analoge 4-Aminochinoline, z.B. Amodiaquine [7-Chlor-4-(3'-dimethylaminomethyl-4'-hydroxy-phenylamino)-chinolin] und seine Säureadditionssalze, wie Hydrochlorid, sowie insbesondere auch Chinin und seine Analoga, z.B. Mefloquine [(2-Piperidyl)-(2,8-bis-trifluormethyl-4-chinolyl)-carbinol] und Halofantrine [(2-Dibutylaminoethyl)-(1,3-dichlor-6-trifluormethyl-9-phenanthrolyl)-carbinol], alle in Form der entsprechenden freien Base oder eines pharmazeutisch brauchbaren Säureadditionssalzes. Weitere Blut-Schizontozide sind beispielsweise gewisse natürliche Antibiotika, wie Tetracyclin, Sulfonamide vom Typ Sulfadoxine [N'-(5,6-Diethoxy-4-pyrimidinyl)-sulfanilamid] und Sulfalene [N'-(3-Me- thoxy-2-pyrazinyl)-sulfanilamid], Sulfone vom Typ Dapsone [4,4'-Diamino-diphenylsulfon], Sesquiterpenoide vom Typ Artemisinin (Qinghaosu), Dihydroartemisinin-methylether (Artemether) und Natriumsalz von Dihydroartemisinin-hemisuccinat (Artesumatesodium), sowie Inhibitoren der Dihydrofolatreduktase wie Proguanil [$N^1$-(4-Chlorphenyl)-$N^5$-isopropyldiguanid] und Pyrimethamine [2,4-Diamino-5-(4-chlorphenyl)-6-ethylpyrimidin]. Die normale therapeutische Dosierung aller dieser Verbindungen ist allgemein bekannt. Bei der erfindungsgemässen Verabreichung ist es möglich, die minimalen Dosen der konventionellen Dosierung anzuwenden, welche für den jeweiligen Einsatz, wie Behandlung der akuten Phase oder Nachbehandlung, üblich sind, oder sogar mit Dosen unterhalb der üblichen Dosierungsgrenze auszukommen. Unter Umständen, insbesondere bei resistenten Plasmodium-Stämmen, kann es jedoch zweckmässig sein, die konventionelle Dosierung der chemotherapeutischen Komponente auch in der erfindungsgemässen Kombination einzuhalten, um einen gegenüber konventioneller Therapie wesentlich günstigeren und/oder kürzeren Verlauf der Heilung zu erreichen. Da die Dosierung von allen bekannten Blut-Schizontoziden in etwa derselben Grössenordnung liegt, können stellvertretend für die ganze Kategorie folgende Angaben als Richtlinie dienen (die Mengen beziehen sich auf Wirkstoff als freie Base, aus welchen äquivalente Dosen für Säureadditionssalze errechnet werden müssen): Bei Erwachsenen mit einer akuten mittelstarken Erkrankung von Malaria tropica wird z.B. Chloroquin am Anfang der akuten Phase (am ersten Tag) in einer Tagesdosis von etwa 900 mg oral verabreicht, in nachfolgenden 2 Tagen wird die Tagesdosis auf etwa 300 mg gesenkt; bei Chinin beträgt die äquivalente Tagesdosis etwa 1,8 g oral während der ganzen Behandlung oder, bei parenteraler Verabreichung (i.v. oder per Infusion) bis etwa 1,8 g am ersten Tag und etwa 1,2 g in nachfolgenden Tagen. Die tägliche Dosis wird in etwa 2-4, üblicherweise 3, Einzeldosen aufgeteilt verabreicht, die Behandlung wird bis zum Verschwinden der Symptome fortgesetzt. Für Nachbehandlung und/oder präventive Verabreichung wird eine einmalige Dosis von 300 mg Chloroquin oral einmal pro Woche als ausreichend betrachtet.

Die Wirkstoffe dieser Kategorie werden zweckmässig in den üblichen pharmazeutischen Dosierungsformen, wie Kapseln, Tabletten und Sirup einerseits, bzw. Injektionslösungen andererseits, verwendet.

Ein erfindungsgemäss anzuwendender Eisen(III)-Chelator ist in erster Linie das oben erwähnte Desferrioxamin B als freie Base oder auch als eines seiner bekannten pharmazeutisch brauchbaren Säureadditionssalze, wie Hydrochlorid und insbesondere Methansulfonat (Mesylat), vgl. The Merck Index, Item 2839, Seite 412; 10. Edition, Merck Co., Inc., Rahway, N.J., U.S.A.; 1983. Als Arzneimittel zur erfindungsgemässen Behandlung von Malaria wird es insbesondere in zur parenteralen Verabreichung geeigneter Form, wie z.B. als wässrige Lösung, zur Verfügung gestellt. Vorzugsweise wird das Arzneimittel, insbesondere ein geeignetes pharmazeutisch brauchbares Säureadditionssalz von Desferroxamin B, z.B. das Mesylat, in einer zur parenteralen Verabreichung von etwa 0,2 bis 5 g täglich, vor allem von etwa 0,2 bis etwa 1,5 g und in erster Linie etwa 0,5 bis etwa 1,0 g täglich, zur Verfügung gestellt. Die Dosierung richtet sich nach dem Gewicht und individuellem Zustand des Patienten und vor allem der Empfindlichkeit des Krankheitserregers; besonders zweckmässig ist die Verabreichung von zweimal täglich 0,5 g, welche bis zum Verschwinden aller Symptome währen 1-2 Wochen fortgesetzt wird. In Anfangsstadien, und besonders bei Anfällen kann diese Tagesdosis bis auf das Vierfache erhöht werden.

Geeignete Formulierungen zur Behandlung von Malaria sind insbesondere 2,5 bis etwa 20%ige, vorzugsweise etwa 5 bis etwa 10%ige, wässrige Lösungen von pharmazeutisch brauchbaren Säureadditionssalzen von Desferrioxamin B, wobei diese Lösungen unmittelbar oder kurz vor der Verabreichung aus entsprechenden Trockenampullen, oder aus anderen stabilen und steril gelagerten Formulierungen des Salzes und aus destilliertem oder entmineralisiertem Wasser hergestellt werden. Beispielsweise können 10%ige oder selbst geringer konzentrierte Lösungen von Desferrioxaminmesylat hergestellt werden unter Verwendung handelsüblicher 500 mg Trockenampullen von DESFERAL® (Warenzeichen der Ciba-Geigy AG) und zum Beispiel destilliertem Wasser oder physiologischer Natriumchloridlösungen. Derartige Lösungen können parenteral verabreicht werden, z.B. durch intramuskuläre Injektion, z.B. in den Glutaeus maximus, oder in die vorderen Oberschenkelmuskel oder den Deltoideusmuskel, sowie auch durch intravenöse oder subkutane Injektion oder durch Infusion. Geeignet insbesondere zur Selbstverabreichung oder zur Verabreichung durch nicht geschulte Personen, z.B. durch Gesundheitshelfer in Entwicklungsländern, ist eine langsame subkutane Injektion, z.B. in die abdominale Haut, unter Verwendung einer tragbaren Infusionspumpe und einer flexibel befestigten Injektionsspritze, über den Verlauf mehrerer Stunden einmal oder zweimal täglich oder kontinuierlich während des Tages oder der Nacht, derart, wie sie zur bekannten Behandlung von Krankheiten wie Thalassämie, ausgeübt wird.

Die Herstellung von Desferrioxamin B und von pharmazeutisch brauchbaren Salzen, insbesondere dem Hydrochlorid, wird in der DE-PS 1 186 076 beschrieben. Darüber hinaus werden Salze, wie das bereits genannte Mesylat (Methansulfonat), Sulfat und Tartrat, in der FR-PS 1 898 M beschrieben. In dieser Veröffentlichung wird auch die Herstellung pharmazeutischer Formulierungen, z.B. von Trockenampullen, Suppositorien und Kapseln, die solche Salze enthalten, beschrieben. Weitere geeignete Salze sind solche, wie sie in der DE-PS 1 186 076 beschrieben werden, einschliesslich beispielsweise solcher mit Phosphorsäuren, Essigsäure, Glykolsäure, Milchsäure, Bernsteinsäure, Maleinsäure, Fumarsäure, Apfelsäure, Weinsäure, Zitronensäure, Ethansulfonsäure und Hydroxyethansulfonsäure.

Ein erfindungsgemäss anzuwendender Eisen(III)-Chelator ist ebenfalls das oben erwähnte Desferri-ferrithiocin und dessen Derivate, wie insbesondere pharmazeutisch brauchbare Salze, vor allem Alkalimetall- und Erdalkalimetallsalze, und pharmakologisch wirksame Analoga, wie insbesondere Niederalkanester, welche in der EP-PS 0045281 beschrieben sind.

**0214933**

Geeignete Formulierungen von Wirkstoffen dieses Typs sind solche zur enteralen, wie oralen, sowie parenteralen, wie subkutanen, Verabreichung, wobei sie den Wirkstoff allein oder im Gemisch mit einem pharmazeutisch anwendbaren Trägermaterial enthalten. Die Dosierung ist etwa dieselbe wie die oben angegebene, auch sie hängt vom Alter, Gewicht und individuellem Zustand des Kranken, von der Empfindlichkeit des Erregers, sowie von der Applikationsweise ab. - Die pharmazeutischen Präparate enthalten von etwa 10 % bis etwa 95 %, vorzugsweise von etwa 20 % bis etwa 90 % (Gewicht/Gewicht) des Wirkstoffes bei festen Formen, von etwa 5 % bis etwa 20 %, vorzugsweise etwa 10 % bei flüssigen Applikationsformen, und können in Dosiseinheitsform, wie Dragées, Tabletten, Kapseln, Suppositorien oder auch Ampullen und Trockensubstanz-Ampullen, vorliegen.

Ein erfindungsgemässes Kombinationspräparat kann zweckmässig aus zwei individuellen, je eine der beiden erwähnten Komponenten enthaltenden pharmazeutischen Zusammensetzungen in einem synergistisch wirkenden Verhältnis bestehen. Diese Alternative ist besonders dann angebracht, wenn jede der beiden Komponenten in einer anderen Darreichungsform optimal wirkt. So erreicht beispielsweise das als Wirkungskomponente A bevorzugte Desferrioxamin B oder ein Säureadditionssalz davon seine volle Wirksamkeit nur bei parenteraler Verabreichung, wogegen aber die bevorzugte Wirkungskomponente B, beispielsweise Chloroquin, für parenterale Verabreichung nicht geeignet ist und bei oraler Gabe viel weniger Nebenwirkungen aufweist. Ein derartiges Kombinationspräparat besteht dann vorzugsweise aus zwei aufeinander in einem obligatorischen Schema abgestimmten Sätzen von Dosierungseinheiten enthaltend je eine Komponente. Die Verabreichung selbst muss dabei nicht immer gleichzeitig erfolgen: so kann beim Einhalten einer festen Gesamtdosierung die Tagesdosis einer Komponente in eine andere Anzahl von Einzeldosen als die der anderen aufgeteilt werden, beispielsweise wird, wenn nötig, ein Injektionspräparat 3 mal täglich in Kombination mit je 2 Tabletten 4 mal täglich verabreicht, entsprechend der Pharmakokinetik beider Komponenten. - Vorzugsweise werden jedoch, wo immer möglich, beide erfindungsgemässen Komponenten in einer gemeinsamen pharmazeutischen Zusammensetzung kombiniert, wie in einer konventionellen Injektionslösung oder vorzugsweise in einer üblichen oralen Darreichungsform, Präparate in Form von Dosierungseinheiten werden dabei besonders bevorzugt. Alle solche pharmazeutischen Zusammensetzungen werden gemäss den geläufigen allgemeinen Regeln und Rezepturen der Pharmazie formuliert und verarbeitet.

Ein synergistisch wirkendes Verhältnis der Komponenten ist ein solches, bei dem die Kombination beider Wirkstoffe eine stärkere Wirkung aufweist, als es einem additiven Beitrag beider Komponenten entsprechen würde. Ein derartiges synergistisches Verhältnis kann in breiten Grenzen auftreten, wobei sein Optimum unter anderem von der spezifischen Wahl der Wirkungskomponenten und in erster Linie von der jeweiligen Empfindlichkeit (oder Resistenz) eines Plasmodium-Stammes gegenüber jeder Komponente abhängig ist. Dieses optimale Verhältnis lässt sich für jeden individuellen Fall und Kombination durch einfache, allgemein bekannte Testmethoden feststellen. Da in den endemischen Zentren von Malaria vorwiegend Parasitenstämme von gleicher, oder mindestens gleichartiger Resistenz vorkommen, hat eine solche Bestimmung für die ganze Region meistenfalls eine allgemeine Bedeutung. Vorzugsweise sind beide Komponenten im erfindungsgemässen Kombinationspräparat im Verhältnis von etwa 4:1 bis etwa 1:4, insbesondere von etwa 2:1 bis etwa 1:2 vertreten, wobei das Verhältnis auf Gewichtsmengen der Wirkstoffe oder vorzugsweise auf eine vergleichbare Wirkungsbasis (wie $IC_{50}$ für ein bestimmtes Plasmodium) bezogen wird.

Beispielsweise kann ein Kombinationspräparat für getrennte Verabreichung jeder Wirkungskomponente aus einer etwa 5-15%igen (G/V), vorzugsweise etwa 10-12%igen, Injektionslösung von Desferrioxamin B (als Mesylat) einerseits und Tabletten oder Dragées zu je 100-300 mg, vorzugsweise etwa 150 mg, Chloroquin andererseits bestehen, welche man als Tagesdosis im Normalfall im Verhältnis von 5-10 ml der Injektionslösung (entsprechend etwa 250-1500 mg, vorzugsweise etwa 500-1000 mg des Eisenchelators) zu 1-3 Tabletten (entsprechend etwa 100-900 mg, vorzugsweise etwa 250-500 mg des Schizontozids) aufgeteilt in 2-6, vorzugsweise 2-4 Portionen einem Erwachsenen verabreicht; in schweren Fällen und insbesondere am Anfang der akuten Phase kann die Dosierung vorübergehend verdoppelt werden. Bei Kindern werden niedrigere Dosen, dem Körpergewicht entsprechend, eingesetzt. Anstatt Mesylat können auch andere geeignete Säureadditionssalze von Desferrioxamin B, oder auch das Desferriferrithiocin und seine Salze verwendet werden; auch Chloroquin kann durch andere Antimalarika, z.B. die oben erwähnten, in wirkungsäquivalenter Dosierung ersetzt werden. Eine pharmazeutische Zusammensetzung enthaltend beide Komponenten gemeinsam ist beispielsweise eine Injektionslösung enthaltend etwa 5-10 % (G/V) Chinin (als Dihydrochlorid) zusammen mit etwa 5-10 % (G/V) Desferrioxoamin B (als Mesylat), welche man in mehreren (2-4, insbesondere 3) Dosen täglich parenteral, vorzugsweise als eine (1-3)-stündige Infusion in einer gesamten Tagesdosis von etwa 5-25, vorzugsweise etwa 10-20 ml verabreichen kann. Eine pharmazeutische Zusammensetzung enthaltend gemeinsam beide Komponenten für orale Verabreichung in Dosierungseinheiten sind beispielsweise Kapseln, Dragées oder Tabletten, welche etwa 100-300 mg Desferriferrithiocin (oder ein Salz davon) zusammen mit etwa 100-300 mg Chloroquin (oder einem analogen Antimalarikum) vorzugsweise in gleichen Gewichtsmengen, enthalten und 1-6 mal, vorzugsweise 2-4 mal, täglich (entsprechend einer Tagesdosis von 100-1600 mg, vorzugsweise 200-900 mg jeder Komponente einem Erwachsenen gegeben werden.

Die pharmazeutischen Präparate der vorliegenden Erfindung werden in an sich bekannter Weise, z.B. mittels konventioneller Misch-, Granulier-, Mahl-, Lösungs- oder Lyophilisierungsverfahren, hergestellt. Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker,
z.B. Lactose, Saccharose, Mannit oder Sorbit, Cellulosepräparate
und/oder Calciumphosphate, z.B. Tricalciumphosphat oder Calciumhydrogenphosphat, ferner Bindemittel, wie Stärkekleister unter
Verwendung z.B. von Mais-, Weizen-, Reis- oder Kartoffelstärke,
Gelatine, Traganth, Methylcellulose, Hydroxypropyl-methylcellulose,
Natriumcarboxymethylcellulose und/oder Polyvinylpyrrolidon,
und/oder, wenn erwünscht, Sprengmittel, wie die obengenannten
Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und
Schmiermittel, z.B. Kieselsäure, Talk, Stearinsäure oder Salze
davon, wie Magnesium- oder Calciumstearat, und/oder Polyethylenglykol. Dragée-Kerne werden mit geeigneten Ueberzügen versehen,
wobei man u.a. konzentrierte Zuckerlösungen, welche gegebenenfalls
arabischen Gummi, Talk, Polyvinylpyrrolidon, Polyethylenglykol
und/oder Titandioxid enthalten, Lacklösungen in geeigneten
organischen Lösungsmitteln oder Lösungsmittelgemischen verwendet.
Den Tabletten oder Dragée-Ueberzügen können Farbstoffe oder
Pigmente, z.B. zur Identifizierung oder zur Kennzeichnung verschiedener Wirkstoffdosen, beigefügt werden.

Die folgenden Beispiele dienen zur Illustration der Erfindung.

<u>Beispiel 1</u>: Kombinationspräparat zur getrennten Verabreichung der
Komponenten.
Komponente A: Trockenampullen zu 250 bzw. 500 mg Wirkstoff [Eisen-
(III)-Chelator]

Ampullen von 5 ml Inhalt werden je mit 2,5 ml einer 10%-igen (G/V)
sterilgefilterten wässrigen Lösung von Desferrioxamin-B-Mesylat
abgefüllt und in üblicher Weise lyophilisiert. Die Injektionslösung
wird durch Zusatz einer entsprechenden Menge (2,5 - 5,0 ml) Wasser
oder physiologischer Lösung (sterilisiert) zubereitet.

In analoger Weise werden auch Trockenampullen mit 500 mg Desferrioxamin-B-Mesylat zur Zubereitung von 5 ml einer 10%-igen (G/V) Injektionslösung hergestellt.

Komponente B: Tabletten zu 150 mg Wirkstoff (Blut-Schizontozid)

Zusammensetzung einer Tablette:

| | |
|---|---|
| Chloroquinsulfat, mikronisiert | 150,0 mg |
| Maisstärke | 50,0 mg |
| Kieselsäure, kollodial | 5,0 mg |
| Gelatine | 5,0 mg |
| Cellulose mikrokristallin | 75,0 mg |
| Natriumcarboxymethylstärke | 20,0 mg |
| Magnesiumstearat | 1,5 mg |
| | 306,5 mg |

Herstellung von 100.000 Tabletten

15 kg Chloroquinsulfat, mikronisiert, und 5,0 kg Maisstärke werden mit 0,5 kg kolloidaler Kieselsäure gemischt und mit einer Lösung von 0,5 kg Gelatine in 5,0 kg destilliertem Wasser (30°C) zu einer feuchten Masse verarbeitet. Diese wird durch ein Sieb von 3 mm Maschenweite getrieben und bei 45°C getrocknet (Wirbelschichttrockner). Das trockene Granulat wird durch ein Sieb von 0,8 mm Maschenweite gedrückt, mit einer im voraus gesiebten Mischung von 7,5 kg mikrokristalliner Cellulose und 2,0 kg Natriumcarboxymethylstärke, sowie mit 0,15 kg Magnesiumstearat gemischt und zu Tabletten von 306,5 mg Gewicht verpresst.

Beispiel 2: Trockenampullen zur gemeinsamen Verabreichung beider Komponenten, enthaltend je 300 mg Desferrioxamin B (als Mesylat) und 300 mg Chinin (als Hydrochlorid).

Ampullen von 7-10 ml Inhalt werden nacheinander mit 3 ml einer sterilgefilterten wässrigen 10%-igen (G/V) Lösung von Chinindihydrochlorid und 2 ml einer sterilgefilterten wässrigen 15%-igen (G/V) Lösung von Desferrioxamin-B-Mesylat abgefüllt und unter üblichen Bedingungen lyophilisiert. (Alternativ kann man 6 ml einer wässrigen Lösung, zubereitet durch Lösen von 100 g jeder Komponente in Wasser und Sterilfiltrieren, abfüllen.)

Die Injektionslösung wird durch Lösen der Trockenmasse unter sterilen Bedingungen durch Zugabe von einer entsprechenden Menge (5-10 ml) sterilem Wasser oder physiologischer Lösung zubereitet.

Beispiel 3: Dragées, enthaltend ca. 100 mg Komponente A [Eisen(III)-Chelator], und 100 mg Komponente B (Blut-Schizontozid), werden folgendermassen hergestellt:

Zusammensetzung eines Dragéekerns:

| | |
|---|---:|
| Komponente A, mikronisiert | 100,0 mg |
| Komponente B, mikronisiert | 100,0 mg |
| Maisstärke | 75,0 mg |
| Tricalciumphosphat | 75,0 mg |
| Polyvinylpyrrolidon K 25 | 15,0 mg |
| Magnesiumstearat | 2,0 mg |
| Natriumcarboxymethylstärke | 33,0 mg |
| | 400,0 mg |

Herstellung von 50 000 Dragéekernen

Die Mischung von 5 kg Komponente A, mikronisiert, 5 kg Komponente B, mikronisiert, 3,75 kg Maisstärke und 3,75 kg Tricalciumphosphat wird mit einer Lösung von 0,75 kg Polyvinylpyrrolidon K 25 in 5 kg destilliertem Wasser im Wirbelschichtverfahren granuliert. Dem bei 45°C getrockneten und durch ein Sieb von 1 mm Maschenweite gedrückten Granulat werden 0,1 kg Magnesiumstearat und 1,65 kg Natriumcarboxymethylstärke zugemischt und zu gewölbten Tabletten à 400 mg verpresst.

Herstellung von 6,6 kg Zuckerdragées

6 kg der Dragéekerne werden in einem Dragierkessel von 45 cm Durchmesser mit einem Zuckersirup (2 Teile Zucker und 1 Gewichtsteil destilliertes Wasser), in dem 1,5 % Polyvinlpyrrolidon K 25 und 1 % Polyethylenglykol 6000 gelöst sind sowie 20 % Talk suspendiert ist, bis zu einem Gewicht von 410 mg portionenweise überzogen, dazwischen wird mit Warmluft von ca. 60° getrocknet. Anschliessend wird Zuckersirup (2 Teile Zucker und 1 Teil Wasser) bis zum Endgewicht von 440 mg portionenweise aufgetragen. Die Dragées werden schliesslich mit einer Lösung von 2 % Carnaubawachs in Trichlorethylen geglänzt.

Als Komponente A wird Desferriferrithiocin (bzw. sein Natriumsalz oder Kaliumsalz in äquivalenten Mengen), als Komponente B Chloroquin (bzw. Amodiaquine, Artemisinin und seine Derivate, Sulfadioxine oder Pyrimethamine in gleichen Mengen) verwendet.

Beispiel 4: Gelatinekapseln enthaltend 125 mg Komponente A
[Eisen(III)-Chelator] und 125 mg Komponente B
(Blut-Schizontozid) werden folgendermassen hergestellt.

Zusammensetzung einer Trockenkapsel

| | |
|---|---:|
| Desferriferrithiocin | 125,0 mg |
| Chloroquindiphosphat | 125,0 mg |
| Milchzucker | 49,0 mg |
| Magnesiumstearat | 1,0 mg |
| | 350,0 mg |

Herstellung von 10 000 Trockenkapseln

1,25 kg Desferriferrithiocin, feinst gemahlen, wird mit 1,25 kg Chloroquin, mikronisiert, innig vermischt und nach Bedarf noch zerrieben; zu diesem Gemisch wird 0,49 kg feinst gemahlener Laktose und 0,01 kg Magnesiumstearat durch ein Sieb zugeben, und homogenisiert. Das Pulver wird gesiebt und in Portionen zu je 350 mg in Gelatinekapseln trocken abgefüllt.

In analoger Weise, wie in den vorangehenden Beispielen 1-4 gezeigt wurde, können auch die anderen in der Beschreibung genannten oder erwähnten Wirkstoffe zu analogen Arzneimitteln verarbeitet werden.

**Versuchsbericht**

**Testmikroorganismen**

**a)** Durch konventionelles Züchten des Stammes Plasmodium
falciparum T96 in einer 2 % (V/V) Suspension von humanen
Erythrozyten wird der Grundorganismus-<u>Zellinie A</u> fortgepflanzt,
welche eine normale Empfindlichkeit gegenüber Chloroquin aufweist.

**b)** Zellinie A des obigen Stammes wird unter analogen Standardbedingungen wie unter a) gezüchtet, jedoch mit Zusatz von immer
ansteigenden Konzentrationen von Chloroquin, bis eine Chloroquin-
resistente <u>Zellinie B</u> resultiert, welche weiterhin in Kulturmedien
mit einer 500 nM-Konzentration von Chloroquin andauernd normales
Wachstum (dieselbe Wachstumsraten wie Zellinie A) aufweist.

**Versuchsanordnung:**

I. Die Züchtung von Testorganismen erfolgt unter den für Züchtung
   von Plasmodien üblichen Bedingungen auf einer 2 % Suspension von
   humanen Erythrozyten, supplementiert mit 50 mg/l Hypoxanthin und
   gegebenenfalls unter Zugabe der getesteten Hemmsubstanz in
   gewünschter Konzentration. Die initiale Parasitämie (= der
   prozentuelle Anteil der zu Beginn von Plasmodien infizierten
   Erythrozyten) beträgt 1-2 %. Das Resultat (Hemmwirkung) wird als
   $IC_{50}$ ausgewertet, d.h. jene Konzentration des Wirkstoffes, die
   eine Senkung der finalen Parasitämie um 50 %, d.h. eine 50%ige
   Hemmung des Wachstums, verursacht.

II. In einer alternativen Versuchsanordnung wird beim Einhalten
    aller übrigen Bedingungen <u>ohne</u> Zugabe von Hypoxanthin und mit
    einer initialen Parasitämie von 0,5 % gearbeitet.

**Vorversuche** (Empfindlichkeit gegenüber Chloroquin)

Zellinie A: Unter Versuchsanordnung I ohne Chloroquin vermehren sich
die Plasmodien innerhalb von 48 Stunden um einen Faktor
von 7-9; $IC_{50}$ = ~50 nM Chloroquin über 2 Zyklen, was
einer normalen Chloroquin-Empfindlichkeit anderer Stämme
entspricht.

Unter Versuchsanordnung II beträgt $IC_{50}$ etwa 10-15 nM
Chloroquin über 2 Zyklen.

Zellinie B: Unter Versuchsanordnung I mit 500 nM-Konzentration von
Chloroquin beträgt der Vermehrungsfaktor wiederum 7-9
(d.h. ist demjenigen von Zellinie A ohne Chloroquin
gleich!); $IC_{50}$ = ~800 nM Chloroquin über 2 Zyklen.


Hauptversuch I (Empfindlichkeit gegenüber Desferrioxamin B)

Zellinie A: Versuchsanordnung I: $IC_{50}$ = 17 ± 3 µM Desferrioxamin B
über 2 Zyklen.

Versuchsanordnung II: $IC_{50}$ = 5 ± 2 µM Desferrioxamin B
über 2 Zyklen.

Zellinie B: a) ohne Chloroquin-Zusatz im Testmedium
Versuchsanordnung I: $IC_{50}$ = 6 ± 1,5 µM Desferrioxamin B
über 2 Zyklen.

Versuchsanordnung II: $IC_{50}$ = 2 ± 1 µM Desferrioxamin B
über 2 Zyklen.

b) mit Zugabe von Chloroquin in einer
400 nM-Konzentration (d.h. unter der Resistenzschwelle)
Versuchsanordnung I: $IC_{50}$ = 3 ± 1 µM Desferrioxamin B
über 2 Zyklen.

Versuchsanordnung II: $IC_{50}$ = 0,6-0,8 ± 0,1 µM
Desferrioxamin B über 2 Zyklen.


Hauptversuch II:

Mit Plasmodium falciparum der Zellinie A (Chloroquin-empfindlich) in
Versuchsanordnung I sind die folgenden Hemmkonzentrationen
festzustellen:

Chloroquin $IC_{20}$ = 10 nM

Desferrioxamin B (Mesylat) $IC_{20}$ = 9 µM

Chloroquin, 10 nM und Desferrioxamin B (Mesylat), 9 µM, kombiniert
bewirken eine 70-100%-ige Wachstumshemmung der obigen Zellinie.


Analoge Resulte werden auch mit Sulfadoxine und Pyrimethamine
anstelle von Chloroquin erhalten.

## Patentansprüche

1. Ein Kombinationspräparat bestehend aus einem Eisen(III)-Chelator der Klasse umfassend Desferrioxamin B und Desferriferrithiocin, sowie ihre pharmakologisch wirksamen Derivate, als Komponente A einerseits und einem konventionellen Antimalarikum der Kategorie von Schizontoziden als Komponente B andererseits, welche Komponenten jede in einer separaten pharmazeutischen Zusammensetzung, oder beide zusammen in einer gemeinsamen pharmazeutischen Zusammensetzung, gegebenfalls unter Beimischung von mindestens einem pharmazeutischen Trägermaterial vorliegen und zueinander in einem synergistisch wirksamen Mengenverhältnis stehen.

2. Kombinationspräparat gemäss Anspruch 1, worin Komponente A in Form einer Injektionslösung und Komponente B in einer oralen Darreichungsform vorliegen.

3. Kombinationspräparat gemäss Anspruch 1, worin beide Komponenten gemeinsam in einem synergistisch wirksamen Verhältnis als eine pharmazeutische Zusammensetzung vorliegen.

4. Kombinationspräparat gemäss Anspruch 3 für parenterale Darreichung.

5. Kombinationspräparat gemäss Anspruch 3 für orale Darreichung.

6. Kombinationspräparat gemäss einem der Ansprüche 1-5 enthaltend als Komponente A Desferrioxamin B in Form von freier Base oder eines pharmazeutisch verwendbaren Säureadditionssalzes.

7. Kombinationspräparat gemäss Anspruch 5 enthaltend als Komponente A Desferriferrithiocin oder ein pharmazeutisch verwendbares Alkalimetall- oder Erdalkalimetallsalz davon.

8. Kombinationspräparat gemäss einem der Ansprüche 1-7, enthaltend als Komponente B Chloroquin in Form von freier Base oder eines pharmazeutisch verwendbaren Säureadditionssalzes.

9. Kombinationspräparat gemäss einem der Ansprüche 1-7, enthaltend als Komponente B Chinin in Form von freier Base oder eines pharmazeutisch verwendbaren Säureadditionssalzes.

10. Kombinationspräparat gemäss einem der Ansprüche 1-9, worin das synergistische Verhältnis etwa 4:1 bis etwa 1:4, bezogen auf Gewichtsmengen, beträgt.

11. Verwendung eines Kombinationspräparates gemäss einem der Ansprüche 1-10 zur Behandlung von Malaria tropica, welche durch Chloroquin-restistente Stämme von Plasmodium falciparum verursacht wird.

12. Verfahren zur Herstellung eines Kombinationspräparats gemäss einem der Ansprüche 1-10 durch konventionelles pharmazeutisches Verarbeiten dessen Komponeten.

FO 7.4 SY/eg*